# EUROPÄISCHE PATENTANMELDUNG

(11) **EP 1 187 052 A2**
(43) Veröffentlichungstag der Anmeldung: **13.03.2002**
(21) Anmeldenummer: 01118466.0
(22) Anmeldetag: 01.08.2001
(51) Int. Cl.: G06F 19/00

(54) **Verfahren zur Erstellung von Synthesepfaden**

(30) Priorität: 06.09.2000 DE 10043853
(71) Anmelder: MERCK PATENT GmbH, 64293 Darmstadt (DE)
(72) Erfinder: Bley, Klemens Dr., 64297 Darmstadt (DE)
(74) Vertreter: Weber, Dieter, Dr.

(57) **Zusammenfassung**

Die vorliegende Erfindung betrifft ein Verfahren zur Erstellung von Synthesepfaden in einer Vielzahl von potentiell miteinander verknüpfbaren Reaktionen. Um ein Verfahren zur Erstellung von Synthesepfaden zu schaffen, welches weitgehend automatisch eine Kette oder auch ein verzweigtes Netzwerk bzw. einen Baum aus verschiedenen Reaktionen liefert, aus welchen alle in dem gespeicherten System bekannten Synthesepfade unmittelbar ablesbar oder darstellbar sind, die zu einem gegebenen Produkt führen oder von einem vorgegebenen Edukt ausgehen werden für das Verfahren die folgenden Schritte vorgeschlagen:.
a) Aufbau einer ersten Datenbank mit den für einen Anwendungszweck gewünschten Edukten und Produkten,
b) Zuordnen eines eindeutigen Codes zu jedem der Edukte und Produkte und Speichern der Codes unter Zuordnungen in der ersten oder einer weiteren Datenbank,
c) Aufbau einer zweiten Datenbank mit einer möglichst großen Anzahl von Reaktionen, in denen die gespeicherten Edukte und/oder Produkte auftreten,
d) Auswahl eines oder mehrerer Edukte oder Produkte,
e) Durchsuchen der zweiten Datenbank nach allen Reaktionen, in denen das bzw. die ausgewählten Edukte und/oder Produkte vorkommen, in einem ersten Durchmusterungsschritt und Zwischenspeichern dieser Reaktionen,
f) Durchsuchen der zweiten Datenbank nach allen Reaktionen, in welchen die Edukte und/oder Produkte der Reaktionen auftreten, welche in der ersten Durchmusterung gefunden und zwischengespeichert wurden und Zwischenspeicherung der dabei erhaltenen Reaktionen,
g) gegebenenfalls Fortsetzung der Durchmusterung in weiteren Schritten, um die Reaktionen zu erhalten, in welchen die zusätzlichen der vorangegangenen Stufe ermittelten Edukte und/oder Produkte erscheinen, und
h) Wiedergabe mindestens einer Reaktionskette und/oder eines Reaktionsbaumes, welche sich über die erhaltenen Reaktionen der verschiedenen Durchmusterungsstufen erstrecken und welche das oder die ausgewählten Edukte bzw. Produkte enthalten.

## Beschreibung

Die vorliegende Erfindung betrifft ein Verfahren zur Erstellung von Synthesepfaden in einer Vielzahl von potentiell miteinander verknüpfbaren Reaktionen. In der chemischen Industrie steht der Fachmann des öfteren vor dem Problem, zur Durchführung eines Verfahrens oder zur Verbesserung von Verfahren bestimmte Produkte oder Zwischenprodukte herstellen zu müssen, ohne daß diese Produkte ohne weiteres und schnell bzw. in der gewünschten Qualität auf dem Markt erhältlich sind. Bei der Vielzahl der inzwischen bekannten chemischen Substanzen sind aber dem Fachmann keineswegs die denkbaren Wege zur Synthese irgendeines gegebenen Produktes geläufig. Zwar gibt es unter Umständen Nachschlagewerke, in denen man Angaben über die Herstellung eines bestimmten Produktes finden kann, dabei ist jedoch völlig offen, ob der betreffende Weg auch in der Praxis sinnvoll, insbesondere wirtschaftlich sinnvoll ist und welche Nachteile gegebenenfalls damit verbunden sind. Weiterhin ist es für den Fachmann oft nicht abzusehen, welche möglichen Folgereaktionen ablaufen können, wenn er ein bestimmtes Produkt hergestellt hat, auch wenn es sich dabei um ein Neben- oder Abfallprodukt handeln mag.

Die vorliegende Erfindung will hier Abhilfe schaffen, indem sie dem Fachmann bei Bedarf eine vollständige Übersicht über alle denkbaren bzw. bekannten Synthesepfade gibt, die zu einem bestimmten Produkt führen oder von einem Edukt ausgehen können. Dabei kann dieses Produkt auch lediglich ein Zwischenprodukt für weitere chemische Reaktionen sein, welches aber nicht ohne weiteres zur Verfügung steht.

Der vorliegenden Erfindung liegt angesichts dieses Standes der Technik die Aufgabe zugrunde, ein Verfahren zur Erstellung von Synthesepfaden zu schaffen, welches weitgehend automatisch eine Kette oder auch ein verzweigtes Netzwerk bzw. einen Baum aus verschiedenen Reaktionen liefert, aus welchen alle in dem gespeicherten System bekannten Synthesepfade unmittelbar ablesbar oder darstellbar sind, die zu einem gegebenen Produkt führen oder von einem vorgegebenen Edukt ausgehen.

Gemäß der Erfindung werden also sowohl alle denkbaren Edukte und Produkte als auch alle Reaktionen aus diesen Edukten und Produkten sorgfältig erfaßt und codiert, das heißt mit einer eindeutigen Bezeichnung bzw. Kennung gespeichert. Aufgrund der codierten Speicherung sowohl der potentiellen Produkte und Edukte als auch der entsprechenden Reaktionen werden in einer ersten Durchmusterung der Datenbank alle Reaktionen erfaßt, bei denen z. B. auf der Produktseite das ausgewählte Produkt erscheint. Diese Reaktionen enthalten darüber hinaus Hinweise auf bzw. Codes der entsprechende(n) Edukte, die in einem nächstfolgenden Durchmusterungsschritt als Produkte anderer Reaktionsgleichungen gesucht werden. Auch hierzu werden wiederum die Reaktionen und die darin enthaltenen Edukte ermittelt, so daß schrittweise immer mehr Edukte erfaßt werden, deren Reaktion zu Zwischenprodukten führt, die wiederum in Reaktion mit anderen Produkten bzw. anderen Zwischenprodukten letztlich zu dem gesuchten Produkt führen. Da im allgemeinen Reaktionen zwischen durchaus unterschiedlichen Kombinationen von Edukten zu ein und demselben Produkt führen können, ergibt sich allgemeinen ein verzweigtes Netzwerk oder eine Baumstruktur aus Reaktionen, wobei der Stamm durch das gesuchte Produkt (oder Edukt) gebildet wird und die Äste und Zweige der Baumstruktur Produkten bzw. Zwischenprodukten entsprechen, während alle Verzweigungspunkte und Verbindungen Reaktionen entsprechen.

Auf diese Weise erhält man mindestens eine, im Regelfall jedoch mehrere Reaktionsketten, die außerdem stark miteinander vernetzt sind, was bedeutet, daß man von einer Reaktionskette auf eine andere wechseln kann, um zu dem ausgewählten Produkt zu gelangen. Dies kann unter Umständen dann zweckmäßig sein, wenn man unerwünschte Zwischenprodukte entlang einer gegebenen ersten Reaktionskette vermeiden will oder wenn dies aus anderen Gründen zweckmäßig erscheint, wie anhand der bevorzugten Ausführungsformen und der Darstellung eines Ausführungsbeispiels noch deutlich werden wird.

Zweckmäßigerweise werden die Reaktionen in der Weise codiert gespeichert, daß ihr Code auch die Codes der an der Reaktion beteiligten Edukte und Produkte enthält. Auf diese Weise kann man unmittelbar an den Reaktionen bzw. den Codierungen der Reaktion ablesen, welche Edukte und Produkte an ihr beteiligt sind. Dies erleichtert besonders die Erfassung aller Reaktionen, an denen z. B. ein bestimmtes Produkt (oder Edukt) beteiligt ist. Die Reaktionen brauchen dann lediglich daraufhin untersucht zu werden, ob sie z. B. auf ihrer Produktseite die Codierung des betreffenden Produktes enthalten. Falls ja, ist die Reaktion Teil einer gewünschten Reaktionskette, falls nein, kann sie für den vorgesehenen Fall ausgesondert werden.

Zweckmäßig ist es außerdem, wenn den Reaktionen und/oder den Edukten oder Produkten jeweils einer oder auch mehrere Bewertungsparameter zugeordnet werden, die es durch Setzen eines Auswahlkriteriums für den Parameterwert ermöglichen, eine Selektion dahingehend vorzunehmen, ob der Parameter für eine Reaktion oder ein Produkt einen vorgegebenen Parameterwert (gegebenenfalls auch eine vorgegebene Summe oder ein Produkt aus Parameterwerten) unterschreitet oder überschreitet. Bei der Darstellung von Reaktionsketten können entsprechende Reaktionen und Produkte, die einen entsprechenden Bewertungsmaßstab unter- bzw. überschreiten, jeweils so gekennzeichnet werden, daß die Unter- bzw. Überschreitung des Bewertungsmaßstabes erkennbar wird, beispielsweise durch farbige Unterlegung.

In der bevorzugten Ausführungsform der Erfindung werden auch die Parameter als Teil der Codes gespeichert. Beispielsweise könnte ein Code eine mehrstellige Zahl sein, welche zum einen eine chemische Substanz kennzeichnet, wobei außerdem mehrere Stellen der Zahl für Parameter reserviert sind, die Zahlenwerte zwischen z. B. 1 und 10 oder 0 und 1 annehmen könnten.

Parameter, die zur Bewertung von Produkten oder Reaktionen herangezogen werden, können insbesondere Maßzahlen für die folgenden Eigenschaften umfassen: Toxizität, Reaktionsbedingungen, apparativer Aufwand, Ausbeute, Kosten der Herstellung und/oder Beschaffung, Stabilität der Edukte/Produkte und Handhabbarkeit der Edukte/Produkte.

Beispielsweise könnte es sein, daß in einer möglichen Reaktionskette eine Reaktion enthalten ist, die nur unter sehr hohem Druck und sehr hoher Temperatur ablaufen kann, was einen entsprechend großen apparativen Aufwand bedeutet. Weiterhin können z. B. Edukte an Reaktionen beteiligt sein, die nicht ohne weiteres oder nicht mit vertretbarem Aufwand mit eigenen Mitteln (gemäß einer entsprechenden Reaktionskette) herstellbar sind, so daß deren Beschaffungskosten ebenfalls als Parameter in die Bewertung eingehen. Eine bevorzugte Vorgehensweise bei der Anwendung des erfindungsgemäßen Verfahrens wird deutlich anhand der folgenden Darstellung eines Ausführungsbeispiels.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Das im Folgenden dargestellte Ausführungsbeispiel beschränkt sich auf einen sehr kleinen Satz von symbolisch wiedergegebenen Substanzen und Reaktionen. Es seien beispielsweise zwölf verschiedene Substanzen (bzw. Moleküle) A bis N gegeben, die gemäß nachstehender Tabelle mit Ziffern von 01 bis 12 codiert und gespeichert werden.

| |
|---|
| A ↔ 01 |
| B ↔ 02 |
| C ↔ 03 |
| D ↔ 04 |
| E ↔ 05 |
| F ↔ 06 |
| G ↔ 07 |
| H ↔ 08 |
| K ↔ 09 |
| L ↔ 10 |
| M ↔ 11 |
| N ↔ 12 |

Die vorstehenden Codes seien in einer Datenbank gespeichert.

Weiterhin seien die folgenden Reaktionen bekannt und gespeichert, an denen die genannten Substanzen A bis N beteiligt sind:

| | | |
|---|---|---|
| I | A + B → | C + D |
| II | D + E → | F + G |
| III | G + B → | H + K |
| IV | A + F → | L + M |
| V | C + L → | N + K |

Die vorstehenden Reaktionen könnten in einem Zahlencode beispielsweise folgendermaßen geschrieben werden:

| | |
|---|---|
| I | 01-02-03-04 |
| II | 04-05-06-07 |
| III | 07-02-08-09 |
| IV | 01-06-10-11 |
| V | 03-10-12-09 |

Die Substanz K sei eine gesuchte Substanz, deren Synthesepfade man ermitteln möchte. Die Substanz K ist mit den Ziffern 09 codiert.

Der Benutzer würde nun beispielsweise in einem entsprechenden Suchfeld entweder die übliche chemische Bezeichnung, für die hier der Buchstabe K steht, eingeben, oder aber den entsprechenden Zahlencode, in diesem Fall 09. Nach dem erfindungsgemäßen Verfahren würde nunmehr in einer Datenbank der gespeicherten Reaktionen eine Suche nach allen Reaktionen beginnen, bei welchen auf der Produktseite der Code 09 auftaucht. An dieser Stelle sei nochmals darauf hingewiesen, daß das obige Beispiel sehr stark vereinfacht ist, indem es nur zweistellige Codes, eine sehr begrenzte Anzahl von chemischen Substanzen und eine sehr begrenzte Anzahl von chemischen Reaktionen verwendet, bei denen außerdem immer nur Reaktionen gespeichert sind, bei welchen aus zwei Edukten zwei Produkte entstehen, so daß klar ist, daß immer die ersten beiden Codes einer Reaktionsgleichung die Edukte wiedergeben und die dritten und vierten Codes die Produkte wiedergeben. Selbstverständlich könnten auch kompliziertere Reaktionen mit mehr Reaktionspartnern und auch unter Angabe von Katalysatoren gespeichert werden, wobei jeweils die Produkt- und die Eduktseite durch eine entsprechende Markierung voneinander getrennt werden müßten.

Bei der Durchsuche der Reaktionsdatenbank stößt das System auf die Gleichungen III und V, die auf der Produktseite den Zifferncode 09, also die Substanz K aufweisen.

Daraus ergeben sich insgesamt folgende mögliche Synthesepfade für die Substanz K, die nachstehend durch die Reihenfolge der Reaktionsgleichungen I bis V wiedergegeben werden.
I-II-III
I-II-IV-V
I-V

Wenn die Edukte F und L nicht unmittelbar als Ausgangstoffe zur Verfügung stehen, weil sie z. B. nicht lagerfähig bzw. instabil sind, so entfällt der kürzeste Reaktionspfad I-V.

Wie man erkennt, erhält man eine weitere Verzweigung in den ersten beiden Ketten bei Gleichung II, indem man entweder das Produkt G der Reaktion II zusammen mit B als Edukte der Reaktion III verwendet, um die Produkte H und K zu erhalten, oder aber über zwei weitere Reaktionen IV und V aus der Reaktion II das Produkt F gemäß Reaktionsgleichung IV verwendet, um das entstehende Zwischenprodukt L gemäß Reaktionsgleichung V zu verwenden und dann die Produkte N und K zu erhalten.

Für Fachleute ist es offensichtlich, daß die Zahl der möglichen Synthesepfade im allgemeinen sehr viel zahlreicher ist und daß die Synthesepfade in der Praxis noch sehr viel stärker verzweigt und vemetzt sind, als bei dem hier dargestellten Beispiel. Beispielsweise kann es neben dem Pfad I-V durchaus noch andere unabhängige Wege geben, um zu den Edukten C und L der Gleichung V zu gelangen, die aber hier nicht wiedergegeben sind.

Synthesepfad I-II-III erscheint nach I-V zunächst als der nächstkürzeste und insofern naheliegendste, zumindest wenn man davon ausgeht, daß die Ausgangsedukte A und B sowie E leicht erhältlich und verfügbar sind. Es könnte aber beispielsweise sein, daß das in der Reaktion III entstehende Produkt H sehr störend sein kann, indem es z. B. toxisch ist oder sich sehr schlecht von dem Produkt K trennen läßt. Denkbar wäre auch, daß die Reaktion III nur unter erheblichem apparativen Aufwand zu bewerkstelligen ist.

Dies führt letztlich dazu, daß unter den vorstehenden Annahmen der Synthesepfad I-II-IV-V der effektivste und wirtschaftlich sinnvollste Synthesepfad ist.

Soweit die Auswahl der Synthesepfade nicht für den Fachmann naheliegend und offensichtlich ist, erscheint es zweckmäßig, in die Reaktionscodes und gegebenenfalls auch in die Substanzcodes noch Parameter aufzunehmen, die die Reaktionen und Substanzen nach unterschiedlichen Gesichtspunkten klassifizieren und eine Bewertung in der Auswahl der Reaktionspfade möglich machen. Z. B. könnten toxische Substanzen einen Parameter aufweisen, der dazu führt, daß in der Darstellung dieser Substanzen, d.h. entweder in der Codedarstellung oder in einer sonstigen üblichen Kennung einige Produkte oder Edukte farbig unterlegt oder in anderer Weise hervorgehoben sind. Auch Reaktionen, die z. B. nur bei hohem Druck und/oder hoher Temperatur ablaufen, könnten durch entsprechende farbige Unterlegungen gekennzeichnet werden. Selbstverständlich sind auch einfache Zusatzangaben in Form eines Zifferncodes, einer Abkürzung oder auch einer Klarschrift denkbar, die dem Benutzer kenntlich machen, welche Abschnitte von Reaktionspfaden möglichst vermieden werden sollten.

Schließlich ist es mit dem erfindungsgemäßen Verfahren auch möglich, mit einem entsprechenden Auswertungsprogramm den unter verschiedenen Gesichtspunkten wirtschaftlichsten oder sinnvollsten Synthesepfad automatisch zu generieren und darzustellen, z. B. auf einem Bildschirm oder in Form eines Ausdrucks mit einer Folge von Reaktionsgleichungen und der Angabe der benötigten Ausgangssubstanzen und Verfahrensparameter.

Hierzu müssen nur die einzelnen Parameter entsprechend bemessen und auch im Verhältnis zueinander und im Hinblick auf gewünschte Randbedingungen gewichtet werden. Es kann beispielsweise sein, daß unter bestimmten Betriebsbedingungen die Toxizität eines Produktes von untergeordneter Bedeutung ist, da die Reaktion ohnehin in einem abgeschlossenen System abläuft und die toxische Substanz anschließend zu 100% weiterverwendet wird oder aber in Folgereaktionen sehr leicht neutralisiert werden kann. In einem solchen Fall würde der Toxizität dieser Substanz nur eine geringe Bedeutung beigemessen werden. Dies kann entsprechend codiert in der zugehörigen Reaktionsgleichung vermerkt bzw. gespeichert sein.

Das erfindungsgemäße Verfahren ermöglicht es, in sehr schneller und einfacher Weise einen vollständigen Überblick über alle Synthesepfade zu erhalten, die zu einem bestimmten Produkt führen, und ermöglicht auf diese Weise auch die schnelle Auswahl der praktisch und wirtschaftlich günstigsten Synthesepfade, die auf andere Weise möglicherweise nur schwer zu finden wären. Selbstverständlich kann das System in gleicher Weise auch verwendet werden, um bei einer gegebenen Gruppe von Edukten oder auch bei einem einzelnen Edukt als Ausgangspunkt festzustellen, welche möglichen Endprodukte hieraus zu erzeugen sind.

## Patentansprüche

1. Verfahren zur Erstellung von Synthesepfaden mit den folgenden Schritten:
a) Aufbau einer ersten Datenbank mit den für einen Anwendungszweck gewünschten Edukten und Produkten,
b) Zuordnen eines eindeutigen Codes zu jedem der Edukte und Produkte und Speichern der Codes unter Zuordnungen in der ersten oder einer weiteren Datenbank,
c) Aufbau einer zweiten Datenbank mit einer möglichst großen Anzahl von Reaktionen, in denen die gespeicherten Edukte und/oder Produkte auftreten,
d) Auswahl eines oder mehrerer Edukte oder Produkte,
e) Durchsuchen der zweiten Datenbank nach allen Reaktionen, in denen das bzw. die ausgewählten Edukte und/oder Produkte vorkommen, in einem ersten Durchmusterungsschritt und Zwischenspeichem dieser Reaktionen,
f) Durchsuchen der zweiten Datenbank nach allen Reaktionen, in welchen die Edukte und/oder Produkte der Reaktionen auftreten, welche in der ersten Durchmusterung gefunden und zwischengespeichert wurden und Zwischenspeicherung der dabei erhaltenen Reaktionen,
g) gegebenenfalls Fortsetzung der Durchmusterung in weiteren Schritten, um die Reaktionen zu erhalten, in welchen die zusätzlichen der vorangegangenen Stufe ermittelten Edukte und/oder Produkte erscheinen, und
h) Wiedergabe mindestens einer Reaktionskette und/oder eines Reaktionsbaumes, welche sich über die erhaltenen Reaktionen der verschiedenen Durchmusterungsstufen erstrecken und welche das oder die ausgewählten Edukte bzw. Produkte enthalten.

2. Verfahren nach Anspruch 1, **dadurch gekennzeichnet, daß** die Reaktionen in der Weise codiert gespeichert werden, daß ihr Code die Codierungen der an der Reaktion beteiligten Edukte und Produkte enthält.

3. Verfahren nach Anspruch 1 oder 2, **dadurch gekennzeichnet, daß** den Reaktionen und/oder den Edukten und Produkten einer oder mehrere Bewertungsparameter zugeordnet werden, durch Setzen eines Auswahlkriteriums für den Parameter eine Selektion von Reaktionen in einer Kette bzw. einem verzweigten Reaktionsbaum erlauben.

4. Verfahren nach Anspruch 3, **dadurch gekennzeichnet, daß** die Parameter bzw. Parameterwerte als Teil des Codes gespeichert werden.

5. Verfahren nach Anspruch 3 oder 4, **dadurch gekennzeichnet, daß** die Parameter ausgewählt werden als Maßzahlen für eine oder mehrere der folgenden Eigenschaften: Toxizität, Reaktionsbedingungen, apparativer Aufwand, Ausbeute, Kosten der Herstellung und/oder Beschaffung, Stabilität der Edukte/Produkte und Handhabbarkeit der Edukte/Produkte.

6. Verfahren nach einem der Ansprüche 3 bis 5, **dadurch gekennzeichnet, daß** in einem Optimierungsprogramm unter Wichtung der Parameterwerte ein oder mehrere optimierte Synthesepfade ermittelt und dargestellt werden.
